# EUROPEAN PATENT APPLICATION

(11) **EP 4 410 240 A1**
(43) Date of publication of application: **07.08.2024**
(21) Application number: 22874878.6
(22) Date of filing: 27.09.2022
(51) Int. Cl.: A61F 2/24

(54) **HEART VALVE PROSTHESIS CAPABLE OF ANCHORING TO NATIVE VALVE LEAFLET**

(30) Priority: 30.09.2021 CN 202111160970
(71) Applicant: Jenscare Scientific Co., Ltd, Ningbo, Zhejiang 315336 (CN)
(72) Inventor: LV, Shiwen, Hangzhou Bay New District Ningbo, Zhejiang 315336 (CN); CHEN, Zhi, Hangzhou Bay New District Ningbo, Zhejiang 315336 (CN); CHEN, Jinxiong, Hangzhou Bay New District Ningbo, Zhejiang 315336 (CN); ZHANG, Shandong, Hangzhou Bay New District Ningbo, Zhejiang 315336 (CN)
(74) Representative: Weickmann & Weickmann PartmbB
(86) International application number: PCT/CN2022/121560
(87) International publication number: WO 2023/051493

(57) **Abstract**

Provided is a heart valve prosthesis capable of anchoring to a native valve leaflet. The proximal end of a clamping mechanism (21) is connected to a valve stent (1). An anchoring ring (22) is disposed on the clamping mechanism (21). After the valve stent (1) is mounted in place, the native valve leaflet is lifted by the anchoring ring (22), and at least one of the native valve leaflet and chordae tendineae tissue is clamped between the valve stent (1) and a valve clamping and fixing device (2). The anchoring ring (22) clamps the valve stent (1).

## Description

### CROSS-REFERENCE TO RELATED APPLICATION(S)

The present application claims priority to a patent application No. 202111160970.4 filed on Sep. 30, 2021, disclosure of which is incorporated herein by reference.

### TECHNICAL FIELD

The present application relates to the field of medical equipment and, in particular, to a heart valve prosthesis capable of anchoring to a native valve leaflet.

### BACKGROUND

From the perspective of a heart structure, both a mitral valve and a tricuspid valve have special physiological structures, making accurate positioning and fixation of a product challenging. In particular, the position of the mitral valve in a heart and its complex anatomic structure pose great challenges for mitral valve replacement.

In the existing art, most of anchoring techniques use the radial support force of a stent to an atrioventricular valve annulus. The disadvantages of this technique include the risk of compressing the tissue around the valve annulus, leading to potential issues with the stent and compressed tissue affecting an outflow tract. In the long term, as the regurgitation decreases, the ventricular and atrial pressure decreases, and a heart structure is reconstructed. A stent of a large specification may affect the reduction of the size of the valve annulus, thereby affecting the internal structure of the heart.

Furthermore, after replacement of the existing prosthesis, an anterior valve leaflet may be attached to the periphery of a stent, which may seriously affect the blood flow supply of a left ventricular outflow tract, thereby affecting the operation of an aorta.

In summary, although the above techniques have shown some clinical results, there are deficiencies. A new transcatheter heart valve replacement system is urgently needed to solve the above problems.

### SUMMARY

In view of the above and other concepts, the present application is proposed. The main object of the present application is to overcome some problems and deficiencies of the existing art.

In terms of the application of atrioventricular valve surgery, the present application is intended to provide a heart valve prosthesis capable of anchoring to a native valve leaflet for patients with atrioventricular valve lesions requiring interventional treatment. Thus, the problems in the existing art that a heart valve replacement system causes compression on a native valve annulus and a valve leaflet blocks an outflow tract are solved.

According to an aspect of the present application, a heart valve prosthesis capable of anchoring to a native valve leaflet is provided. The heart valve prosthesis includes a valve stent and a valve clamping and fixing device connected to the valve stent. The valve clamping and fixing device includes a clamping mechanism and an anchoring ring. The proximal end of the clamping mechanism is connected to the valve stent. The distal end of the clamping mechanism is free. At least part of the anchoring ring is disposed on the clamping mechanism. The native valve leaflet is configured to be lifted by the anchoring ring after the valve stent is mounted in place. The valve stent and the valve clamping and fixing device are configured to clamp at least one of the native valve leaflet and chordae tendineae tissue between the valve stent and the valve clamping and fixing device. The anchoring ring is configured to clamp the valve stent after the valve stent is mounted in place.

According to an embodiment, the valve stent is provided with an artificial valve leaflet. The artificial valve leaflet may be made of a biological material such as bovine pericardium or porcine pericardium, or may be made of a polymer material.

According to an embodiment, the valve clamping and fixing device has a first form and a second form. The clamping mechanism is configured to capture the native valve leaflet when the valve clamping and fixing device is in the first form. When the valve stent radially expands so that the anchoring ring pulls chordae tendineae and drives the native valve leaflet to lift upwards, the valve clamping and fixing device is in the second form.

According to an embodiment, the clamping mechanism is configured to be located between the native valve leaflet and a ventricular wall when the valve clamping and fixing device is in the first form. Moreover, part of the anchoring ring is configured to be located between the native valve leaflet and the chordae tendineae tissue when the valve clamping and fixing device is in the first form.

According to an embodiment, the valve stent and the clamping mechanism are an integrated structure. Moreover, at least part of the clamping mechanism fits against the outer side of the valve stent in a natural state.

According to an embodiment, the anchoring ring and part of the clamping mechanism are covered with a fabric layer. This design may effectively increase the frictional force of the anchoring ring and the clamping mechanism against the native valve leaflet.

According to an embodiment, the anchoring ring is a closed-ring structure, or the anchoring ring cooperates with the clamping mechanism to form a closed-ring structure.

According to an embodiment, the anchoring ring has at least two connection points with the clamping mechanism. The at least two connection points between the anchoring ring and the clamping mechanism are at different heights. Alternatively, the anchoring ring has at least two connection points with the clamping mechanism and the valve stent, and a connection point between the anchoring ring and the clamping mechanism, and a connection point between the anchoring ring and the valve stent are at different heights.

According to an embodiment, the clamping mechanism includes a single clamping arm. The anchoring ring is connected to the clamping arm and the valve stent. Moreover, the height of the connection point between the anchoring ring and the valve stent is lower than the height of the connection point between the anchoring ring and the clamping arm.

According to another embodiment, in a case where the heart valve prosthesis is configured to treat a mitral valve, and the clamping arm is configured to capture and clamp an anterior valve leaflet when the valve clamping and fixing device is in the first form, and a connection point between the anchoring ring and the valve stent is configured to be located in a posterior valve leaflet region when the valve clamping and fixing device is in the first form.

According to another embodiment, the clamping mechanism includes a first clamping arm and a second clamping arm. The anchoring ring is connected to the first clamping arm and the second clamping arm. Moreover, the first clamping arm and the second clamping arm are configured to capture and clamp different native valve leaflets respectively. Moreover, the diameter of the anchoring ring is smaller than the diameter of the valve stent. Moreover, the diameter of the anchoring ring is smaller than the diameter of a native valve annulus.

According to an embodiment, the valve clamping and fixing device also includes a control filament. The control filament is detachably connected to the first clamping arm and the second clamping arm. Moreover, the control filament is configured to be pulled to enable the first clamping arm and second clamping arm to open to capture the native valve leaflet.

According to another embodiment, the clamping mechanism includes multiple clamping arms. The connection points between the anchoring ring and the multiple clamping arms are at different heights, so that the anchoring ring is in a wavy shape.

According to an embodiment, the distal end of the valve stent is provided with multiple auxiliary support stems. The auxiliary support stems are configured to be located in an atrium after the valve stent is mounted in place. Moreover, at least part of the auxiliary support stems are configured to fit against an atrial wall after the valve stent is mounted in place. When the valve stent is configured to treat a mitral valve, the distance from the free end of the auxiliary support stem of an anterior valve leaflet region to the center point of the valve stent is greater than the distance from the free end of the auxiliary support stem of the posterior valve leaflet region to the center point of the valve stent. The object of the design is to make the whole valve stent fit against the posterior valve leaflet region, so that the valve stent reduces the blocking to a left ventricular outflow tract after implantation.

According to an embodiment, the heart valve prosthesis also includes a sealing device. The sealing device is configured to conform to the physiological structure of a valve annulus. The sealing device includes an adaptive segment and an abutment segment. The adaptive segment includes a first arc segment, a second arc segment, and a leak-proof membrane. The first arc segment is connected to the valve stent. The valve stent provides a fulcrum for the first arc segment. At least part of the first arc segment fits against an atrial wall after the valve stent is mounted in place. The first arc segment is configured to rotate about the fulcrum toward the center of the valve stent to drive the second arc segment to rotate in a same direction as the first arc segment rotates when the atrial wall squeezes the first arc segment.

According to an embodiment, the leak-proof membrane covers the first arc segment and the second arc segment. Moreover, the leak-proof membrane connects the free end of the first arc segment and the free end of the second arc segment.

According to an embodiment, when the heart valve prosthesis is configured to treat the mitral valve, the adaptive segment is located in the anterior valve leaflet region, and the abutment segment is located in the posterior valve leaflet region.

According to an embodiment, the valve stent is provided with barbs in the posterior valve leaflet region for anchoring the native valve leaflet.

According to an embodiment, the anchoring ring is generally in the shape of a sheet, a filament, or a hemp rope.

Compared with the existing art, the advantages and beneficial technical effects of the present application include at least the following.
1. Most traditional valve prostheses rely on a radial support valve annulus to anchor a prosthesis, which may cause compression on a native valve annulus. The anchorage defects are apparent. Furthermore, the existing solution that uses a valve leaflet to anchor a stent cannot solve the blocking impact on a left ventricular outflow tract. In an embodiment of the present application, the clamping mechanism is configured to bring the anchoring ring to the position below the native valve leaflet and further operate to make the anchoring ring located between the chordae tendineae tissue and the native valve leaflet. When the valve stent radially expands, the anchoring ring is tensioned and pulls the chordae tendineae and drives the native valve leaflet to lift upwards to prevent the native valve leaflet from blocking an outflow tract after the valve stent is implanted. At the same time, the valve stent radially expands and cooperates with the anchoring ring so that the native valve leaflet and the chordae tendineae are clamped between the valve stent and the anchoring ring. In this anchoring method, the valve stent may be prevented from greatly radially expanding the native valve annulus, and the native tissue of a heart is prevented from being squeezed and pulled excessively, thereby reducing postoperative complications. Thus, two major technical pain points in the treatment of mitral valve replacement surgery in the existing art are solved, and there is great clinical significance.
2. In the existing art, an anchoring ring plays the role of fixing a valve stent, but cannot solve the blocking impact of a native valve leaflet on a left ventricular outflow tract. Different from the existing art, in an embodiment of the present application, the anchoring ring can not only embrace a valve leaflet, so that the valve stent is fixed to the native valve leaflet, avoiding the radial support to a native annulus. At the same time, the anchoring ring can also pull the chordae tendineae and further drive the native valve leaflet to lift upwards, effectively preventing the native valve leaflet from blocking the outflow tract. There is great clinical significance.
3. Different from the existing art, in an embodiment of the present application, the connection points of the anchoring ring on a valve clamping member are at different heights. Specifically, the height of the anchoring ring in the anterior valve leaflet region is greater than the height of the anchoring ring in the posterior valve leaflet region. In this manner, the anterior valve leaflet can be significantly lifted to avoid blocking the left ventricular outflow tract, and a clamping member can also be prevented from excessively pulling the chordae tendineae in the posterior valve leaflet region, so that the chordae tendineae tissue is protected.
4. In the existing art, in a direction perpendicular to the cross section of the axis in the valve stent, the cross-section area of a sealing device is greater than the cross-section area of a native valve annulus, so that the sealing device can completely cover the entire native valve annulus, and the free edge portion of the sealing device fits against an atrial wall to implement a better leak-proof effect. However, the inner wall surface of the atrium is not very regular and smooth, so that some parts of the atrial wall exert a greater squeezing force on the sealing device. As a result, the sealing device develops wrinkles and gaps, and blood backflow occurs. In addition, after long-term implantation of a prosthesis, due to the reduction of the valve backflow, the atrium may reconstruct and shrink to the size before a lesion. As a result, the atrial wall may exert an excessive squeezing force on the sealing device, the sealing device develops wrinkles and gaps, and blood backflow occurs. Different from the existing art, in an embodiment of the present invention, the sealing device includes an adaptive segment and an abutment segment. The adaptive segment includes a first arc segment, a second arc segment, and a leak-proof membrane. When the first arc segment is squeezed by an atrial wall, the first arc segment rotates about the fulcrum toward the center of the valve stent, and the first arc segment drives the second arc segment to rotate in the same direction and fit against the underside of the native valve leaflet in time to compensate for the position in real time to avoid blood backflow. Moreover, the sealing device may make the entire prosthesis fit against the posterior valve leaflet region to create room for the left ventricular outflow tract as much as possible to avoid blocking the left ventricular outflow tract.

Embodiments of the present application can implement other beneficial technical effects not listed one by one. These other technical effects may be partially described below and may be expected and understood by those skilled in the art after reading the present application.

### BRIEF DESCRIPTION OF DRAWINGS

The preceding features and advantages and other features and advantages of these embodiments and the method for implementing them may become more apparent by reference to the description below in conjunction with drawings, and embodiments of the present application may be better understood.
FIGS. 1a to 1c are diagrams illustrating the overall structures of a heart valve prosthesis according to the present invention.
FIGS. 2a to 2c are diagrams illustrating the structures of a sealing device of the heart valve prosthesis according to the present invention, where FIG. 2c is a bottom view of the sealing device of the heart valve prosthesis according to the present invention.
FIGS. 3a and 3b are diagrams of the heart valve prosthesis after implantation according to the present invention, where FIG. 3b is a principle diagram in which a second arc segment rotates as a first arc segment rotates, and dashed lines refer to a diagram of the rotation of the first arc segment and the second arc segment after the sealing device is squeezed.
FIGS. 4a to 4f are diagrams of the implantation process of an artificial valve prosthesis according to the present invention, where FIG. 4f is a partial enlarged view of FIG. 4e.
FIGS. 5a and 5b are diagrams according to another embodiment of the present invention.
FIGS. 6a and 6b are diagrams according to another embodiment of the present invention.

The names of the parts denoted by the numbers in the drawings are as follows:
- 1: valve stent
- 11: auxiliary support stem
- 12: sealing device
- 121: adaptive segment
- 1211: first arc segment
- 1212: second arc segment
- 1213: leak-proof membrane
- 122: abutment segment
- 13: barb
- 2: valve clamping and fixing device
- 21: clamping mechanism
- 211: clamping arm
- 2111: first clamping arm
- 2112: second clamping arm
- 22: anchoring ring
- 23: fabric layer
- 24: control filament
- 3: delivery device

### DETAILED DESCRIPTION

The details of one or more embodiments of the present application are set forth in the description below of drawings and embodiments. Other features, objects and advantages of the present application may be apparent from these description and drawings and the claims.

It is to be understood that the illustrated and described embodiments are not limited in application to the details of construction and arrangement of the components set forth in the description below or illustrated in drawings. The illustrated embodiments may be other embodiments and can be implemented or executed in various ways. Various examples are provided by way of explanation and not limitation of the disclosed embodiments. Actually, it is apparent to those skilled in the art that various modifications and variations may be made to various embodiments of the present application without departing from the scope or substance disclosed in the present application. For example, features illustrated or described as part of an embodiment may be used with another embodiment to still produce other embodiments. Thus, the present application discloses and covers such modifications and variations within the claims of the present application and the equivalents thereof.

Similarly, it is to be understood that the phrases and terminologies used herein are for the purpose of description and should not be considered as limiting. The use of "including," "comprising," or "having" and variations thereof herein is intended to openly include the items listed thereafter and equivalents thereof, as well as possible additional items.

The present application may be described in more detail with reference to different embodiments and examples of several aspects of the present application.

In the present application, a proximal end refers to an end proximal to an apex, and a distal end refers to an end distal to the apex.

### Detailed embodiment

### Embodiment one

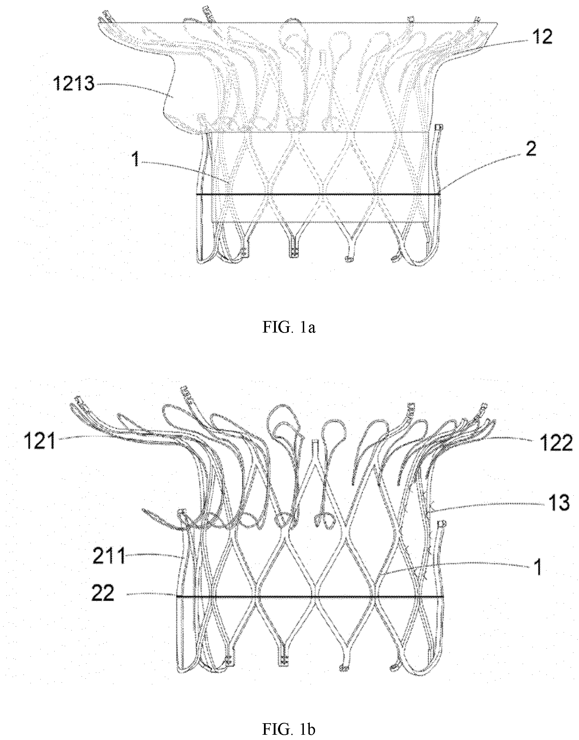

As shown in FIGS. 1a to 1c, in this embodiment, a heart valve prosthesis capable of anchoring to a native valve leaflet includes a valve stent 1 and a valve clamping and fixing device 2 connected to the valve stent 1. The valve clamping and fixing device 2 includes a clamping mechanism 21 and an anchoring ring 22. The proximal end of the clamping mechanism 21 is connected to the valve stent 1. The distal end of the clamping mechanism 21 is free. Moreover, at least part of the anchoring ring 22 is disposed on the clamping mechanism 21. After the valve stent 1 is mounted in place, the native valve leaflet is lifted by the anchoring ring 22, and at least one of the native valve leaflet and chordae tendineae tissue is clamped between the valve stent 1 and the valve clamping and fixing device 2. Moreover, the anchoring ring 22 clamps the valve stent 1.

According to an embodiment, the valve stent 1 is provided with an artificial valve leaflet. The artificial valve leaflet may be made of a biological material such as bovine pericardium or porcine pericardium, or may be made of a polymer material.

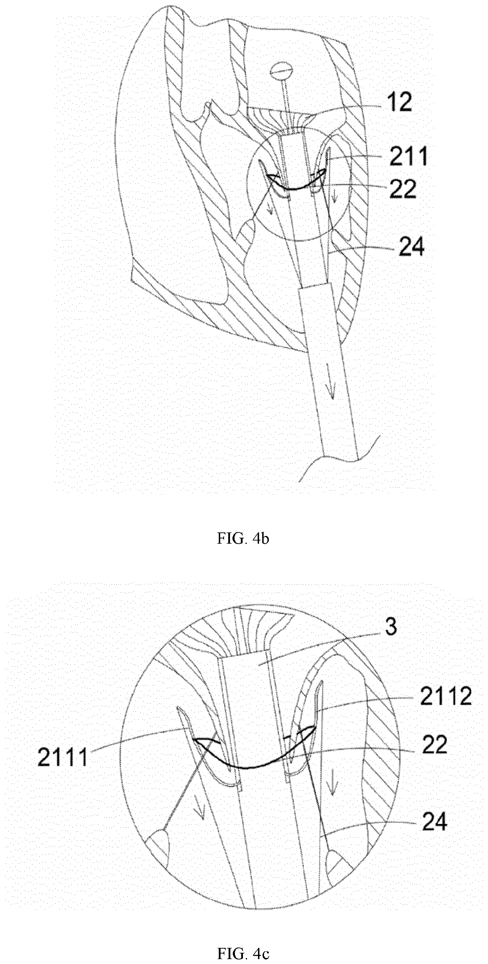

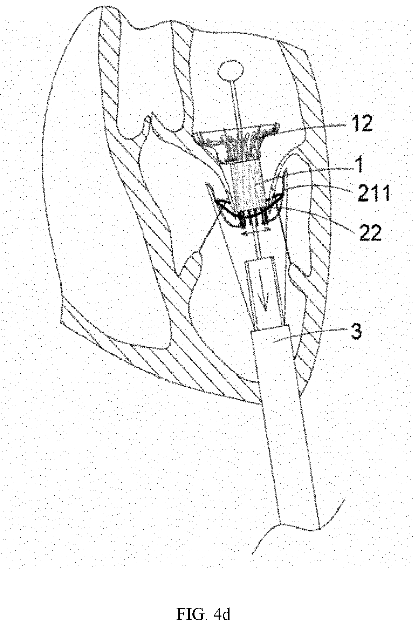

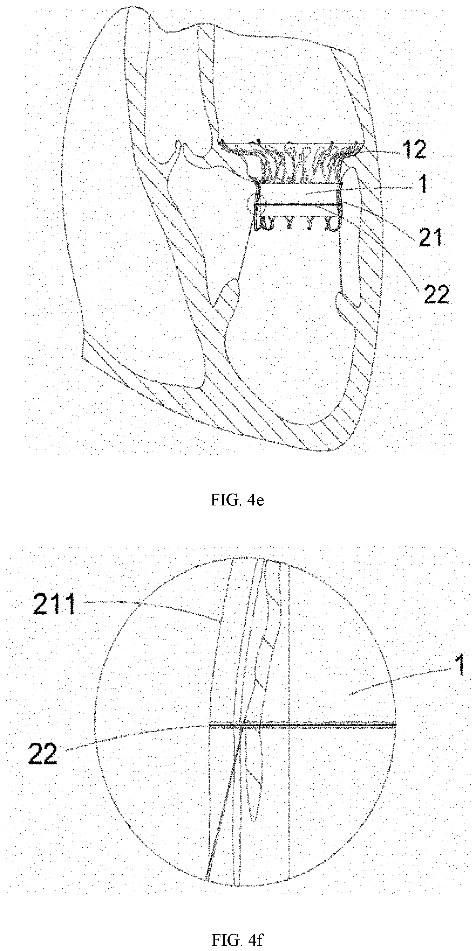

According to an embodiment, the valve clamping and fixing device 2 has a first form and a second form. When the valve clamping and fixing device 2 is in the first form, the clamping mechanism 21 is configured to capture the native valve leaflet, as shown in FIGS. 4b and 4c. When the valve clamping and fixing device 2 is in the second form, the valve stent 1 radially expands so that the anchoring ring 22 pulls chordae tendineae and drives the native valve leaflet to lift upwards, as shown in FIGS. 4d and 4e.

According to an embodiment, when the valve clamping and fixing device 2 is in the first form, the clamping mechanism 21 is between the native valve leaflet and a ventricular wall. Moreover, part of the anchoring ring 22 is between the native valve leaflet and the chordae tendineae tissue.

According to an embodiment, the valve stent 1 and the clamping mechanism 21 are an integrated structure. Moreover, at least part of the clamping mechanism 21 fits against the outer side of the valve stent 1 in a natural state.

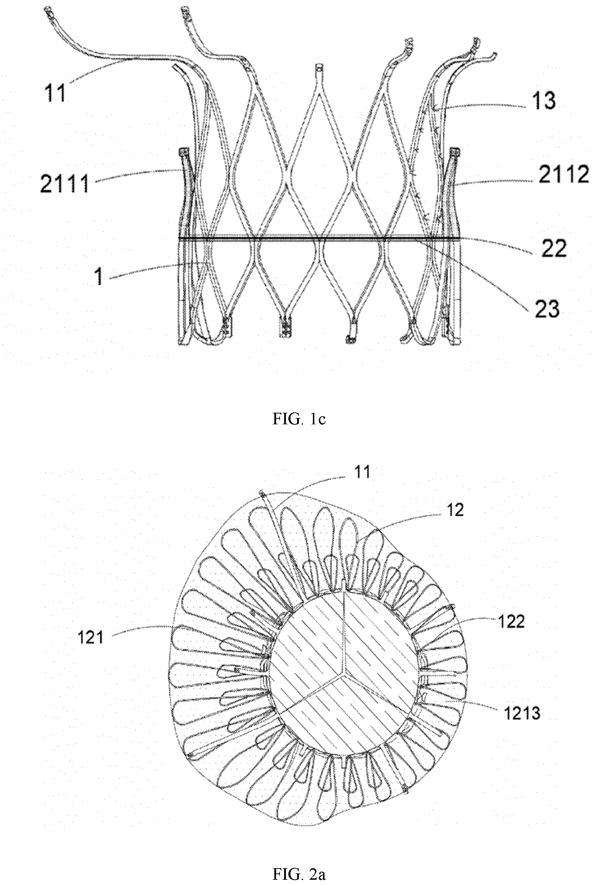

According to an embodiment, the anchoring ring 22 and part of the clamping mechanism 21 are covered with a fabric layer 23. This design may effectively increase the frictional force of the anchoring ring 22 and the clamping mechanism 21 against the native valve leaflet, as shown in FIG. 1c.

According to an embodiment, the anchoring ring 22 is a closed-ring structure, or the anchoring ring 22 cooperates with the clamping mechanism 21 to form a closed-ring structure.

According to an embodiment, the anchoring ring 22 and the clamping mechanism 21 include at least two connection points, and the connection points between the anchoring ring 22 and the clamping mechanism 21 are at different heights.

According to an embodiment, the clamping mechanism 21 includes a first clamping arm 2111 and a second clamping arm 2112. The anchoring ring 22 is connected to the first clamping arm 2111 and the second clamping arm 2112. Moreover, the first clamping arm 2111 and the second clamping arm 2112 are configured to capture and clamp different native valve leaflets respectively. Moreover, the diameter of the anchoring ring 22 is smaller than the diameter of the valve stent 1. Moreover, the diameter of the anchoring ring 22 is smaller than the diameter of a native valve annulus.

According to an embodiment, the valve clamping and fixing device 2 also includes a control filament 24. The control filament 24 is detachably connected to the first clamping arm 2111 and the second clamping arm 2112. Moreover, the control filament 24 is pulled to enable the first clamping arm 2111 and the second clamping arm 2112 to open to capture the native valve leaflet.

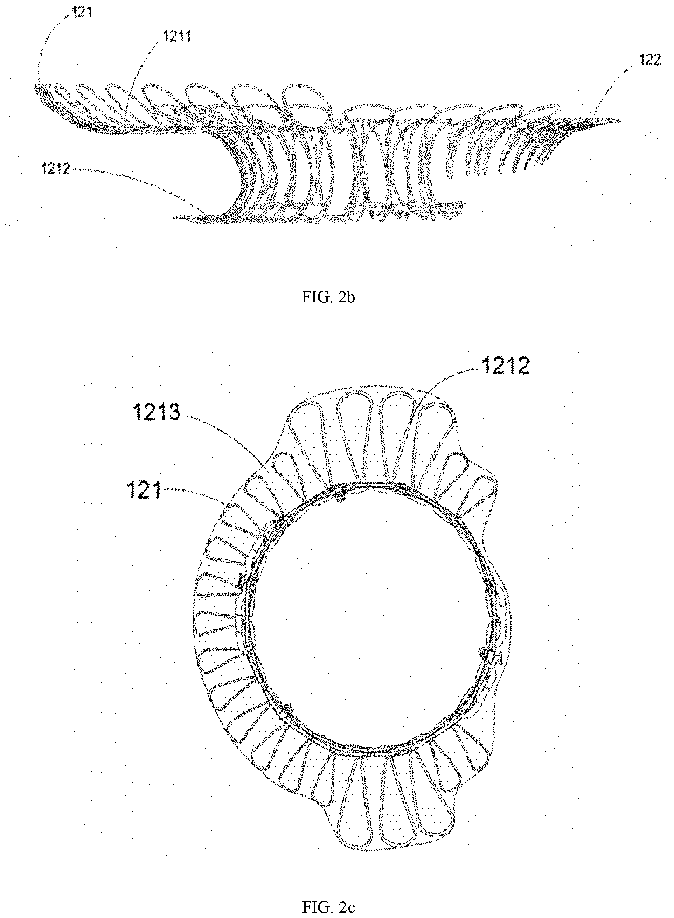

According to an embodiment, the distal end of the valve stent 1 is provided with multiple auxiliary support stems 11. After the valve stent 1 is mounted in place, the auxiliary support stems 11 are located in an atrium. Moreover, at least part of the auxiliary support stems 11 fits against an atrial wall. When the valve stent 1 is configured to treat a mitral valve, the distance from the free end of the auxiliary support stem 11 of an anterior valve leaflet region to the center point of the valve stent 1 is greater than the distance from the free end of the auxiliary support stem 11 of a posterior valve leaflet region to the center point of the valve stent 1, as shown in FIGS. 1c and 2a. The objective of the design is to make the whole valve stent 1 fit against the posterior valve leaflet region, so that the valve stent 1 reduces the blocking to a left ventricular outflow tract after implantation.

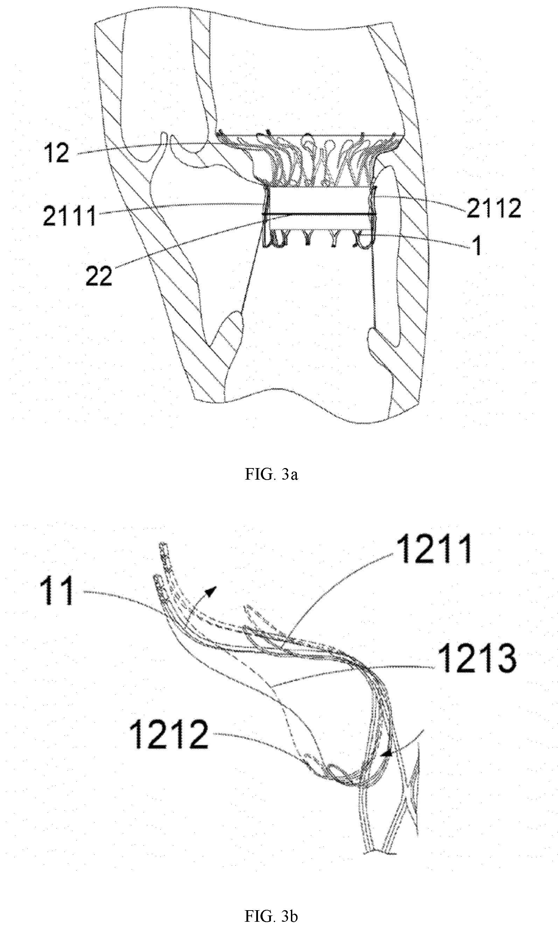

According to an embodiment, the heart valve prosthesis also includes a sealing device 12. The sealing device 12 is configured to conform to the physiological structure of a valve annulus. The sealing device 12 includes an adaptive segment 121 and an abutment segment 122. The adaptive segment 121 includes a first arc segment 1211, a second arc segment 1212, and a leak-proof membrane 1213, as shown in FIGS. 2a to 2c. The first arc segment 1211 is connected to the valve stent 1, and the valve stent 1 provides a fulcrum for the first arc segment 1211. After the valve stent 1 is mounted in place, at least part of the first arc segment 1211 fits against an atrial wall. When the atrial wall squeezes the first arc segment 1211, the first arc segment 1211 rotates about the fulcrum toward the center of the valve stent 1. Moreover, the first arc segment 1211 drives the second arc segment 1212 to rotate in the same direction, as shown in FIGS. 3a and 3b.

According to an embodiment, the leak-proof membrane 1213 covers the first arc segment 1211 and the second arc segment 1212. Moreover, the leak-proof membrane 1213 connects the free end of the first arc segment 1211 and the free end of the second arc segment 1212.

According to an embodiment, when the heart valve prosthesis is configured to treat a mitral valve, the adaptive segment 121 is located in the anterior valve leaflet region, and the abutment segment 122 is located in the posterior valve leaflet region.

According to an embodiment, the valve stent 1 is provided with barbs 13 in the posterior valve leaflet region for anchoring the native valve leaflet, as shown in FIG. 1c.

According to an embodiment, the anchoring ring 22 is generally in the shape of a sheet, a filament or a hemp rope.

According to an embodiment, the heart valve prosthesis also includes a delivery device 3 for delivering it to a surgical site.

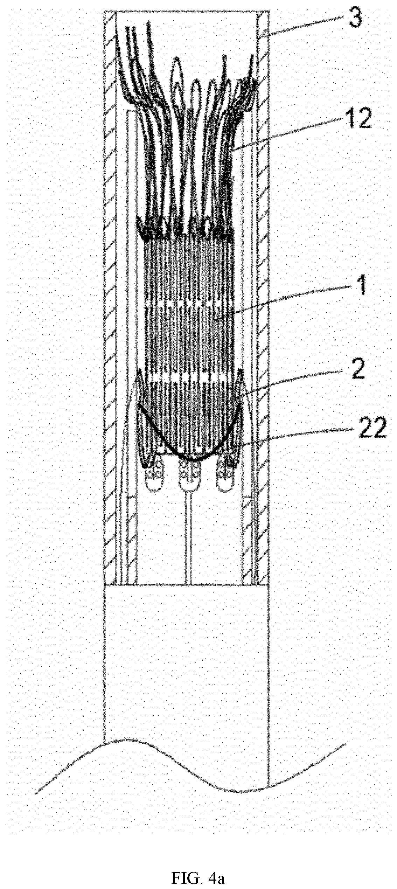

In embodiment one, an exemplary procedure for repairing a mitral valve using a heart valve prosthesis capable of anchoring to a native valve leaflet is below, as shown in FIGS. 4a to 4f.
1. The delivery device 3 is operated from a transapical approach so that the distal portion of the delivery device 3 is located in the left atrium.
2. The delivery device 3 is operated to release the distal portion of the valve stent 1. At this time, the sealing device 12 and the auxiliary support stems 11 are not fully released. The control filament 24 is pulled so that the first clamping arm 2111 and the second clamping arm 2112 are opened to capture the native valve leaflet, as shown in FIGS. 4b and 4c.
3. When the first clamping arm 2111 and the second clamping arm 2112 are located behind an anterior valve leaflet and a posterior valve leaflet respectively, and the anchoring ring 22 is between the native valve leaflet and the chordae tendineae tissue, the delivery device 3 is further operated to make the sealing device 12 and the auxiliary support stems 11 fully released and support in the left atrium.
4. The delivery device 3 is operated to release the proximal portion of the valve stent 1 until the valve stent 1 radially expands fully and cooperates with the anchoring ring 22, so that the native valve leaflet and the chordae tendineae are clamped between the valve stent 1 and the anchoring ring 22 finally. Then the delivery device 3 is evacuated to complete the implantation, as shown in FIGS. 4d to 4f.

### Embodiment two

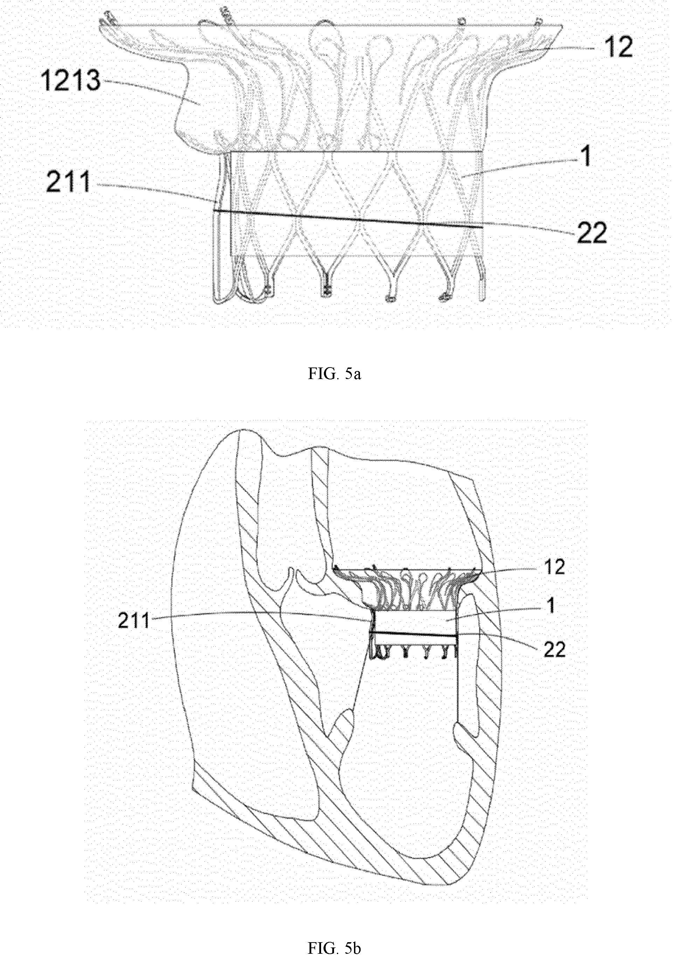

Embodiment two is generally similar to embodiment one. The difference is that the clamping mechanism 21 in this embodiment has only a single clamping arm 211. The anchoring ring 22 is connected to the clamping arm 211 and the valve stent 1, as shown in FIG. 5a.

In this embodiment, the clamping mechanism 21 includes a single clamping arm 211. The anchoring ring 22 is connected to the clamping arm 211 and the valve stent 1. Moreover, the height of the connection point between the anchoring ring 22 and the valve stent 1 is lower than the height of the connection point between the anchoring ring 22 and the clamping arm 211. After the valve stent 1 is mounted in place, the clamping arm 211 captures and clamps the anterior valve leaflet, and the connection point between the anchoring ring 22 and the valve stent 1 is in the posterior valve leaflet region. The object of the design is that the anterior valve leaflet can be significantly lifted to avoid blocking the left ventricular outflow tract, and a clamping member can also be prevented from excessively pulling the chordae tendineae in the posterior valve leaflet region, so that the chordae tendineae tissue is protected, as shown in FIG. 5b.

In this regard, the relevant structure and concept of embodiment two are similar to the relevant structure and concept of embodiment one, and the description is not repeated here.

### Embodiment three

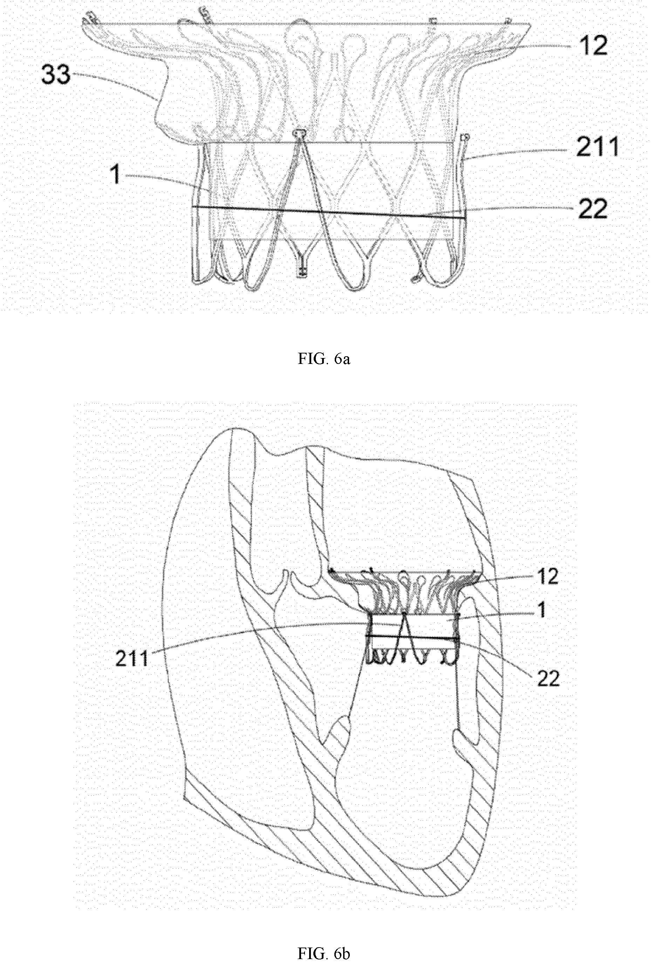

Embodiment three is generally similar to embodiment one. The difference is that in this embodiment, the clamping mechanism 21 includes multiple clamping arms 211, as shown in FIG. 6a.

In this embodiment, the clamping mechanism 21 includes multiple clamping arms 211, and the connection points between the anchoring ring 22 and the multiple clamping arms 211 are at different heights, resulting in a wavy shape of the anchoring ring 22. After the valve stent 1 is mounted in place, the connection point between the anchoring ring 22 and a clamping arm 211 in the anterior valve leaflet region is higher than the connection point between the anchoring ring 22 and a clamping arm 211 in the posterior valve leaflet region. The object of the design is that the anterior valve leaflet can be significantly lifted to avoid blocking the left ventricular outflow tract, and the clamping member can also be prevented from excessively pulling the chordae tendineae in the posterior valve leaflet region, so that the chordae tendineae tissue is protected, as shown in FIG. 6b.

In this embodiment, the perimeter of the anchoring ring 22 is greater than the perimeter of the valve stent 1. Moreover, the perimeter of the anchoring ring 22 is smaller than the perimeter of the native valve annulus.

In this regard, the relevant structure and concept of embodiment three are similar to the relevant structure and concept of embodiment one, and the description is not repeated here.

The foregoing description for example embodiments of the present application are proposed for the purpose of illustration. The foregoing description is not intended to be exhaustive or to limit the present application to the precise arrangement and/or construction disclosed. Apparently, in light of the preceding teachings, many modifications and variations may be made by those skilled in the art without departing from the present invention. The scope and equivalents of the present invention are intended to be defined by the appended claims.

## Claims

1. A heart valve prosthesis capable of anchoring to a native valve leaflet, comprising a valve stent and a valve clamping and fixing device connected to the valve stent, wherein the valve clamping and fixing device comprises a clamping mechanism and an anchoring ring, a proximal end of the clamping mechanism is connected to the valve stent, a distal end of the clamping mechanism is free, and at least part of the anchoring ring is disposed on the clamping mechanism; and the native valve leaflet is configured to be lifted by the anchoring ring after the valve stent is mounted in place, the valve stent and the valve clamping and fixing device are configured to clamp at least one of the native valve leaflet and chordae tendineae tissue between the valve stent and the valve clamping and fixing device, and the anchoring ring is configured to clamp the valve stent after the valve stent is mounted in place.

2. The heart valve prosthesis capable of anchoring to a native valve leaflet according to claim 1, wherein the valve clamping and fixing device has a first form and a second form, wherein the clamping mechanism is configured to capture the native valve leaflet when the valve clamping and fixing device is in the first form; and when the valve stent radially expands so that the anchoring ring pulls chordae tendineae and drives the native valve leaflet to lift upwards, the valve clamping and fixing device is in the second form.

3. The heart valve prosthesis capable of anchoring to a native valve leaflet according to claim 2, wherein the clamping mechanism is configured to be located between the native valve leaflet and a ventricular wall when the valve clamping and fixing device is in the first form, and part of the anchoring ring is configured to be located between the native valve leaflet and the chordae tendineae tissue when the valve clamping and fixing device is in the first form.

4. The heart valve prosthesis capable of anchoring to a native valve leaflet according to claim 1, wherein the valve stent and the clamping mechanism are an integrated structure, and at least part of the clamping mechanism fits against an outer side of the valve stent in a natural state.

5. The heart valve prosthesis capable of anchoring to a native valve leaflet according to claim 1, wherein the anchoring ring has at least two connection points with the clamping mechanism, and the at least two connection points between the anchoring ring and the clamping mechanism are at different heights; or the anchoring ring has at least two connection points with the clamping mechanism and the valve stent, and a connection point between the anchoring ring and the clamping mechanism, and the connection point between the anchoring ring and the valve stent are at different heights.

6. The heart valve prosthesis capable of anchoring to a native valve leaflet according to claim 1, wherein the clamping mechanism comprises a single clamping arm, the anchoring ring is connected to the clamping arm and the valve stent, and a height of a connection point between the anchoring ring and the valve stent is lower than a height of a connection point between the anchoring ring and the clamping arm.

7. The heart valve prosthesis capable of anchoring to a native valve leaflet according to claim 6, wherein in a case where the heart valve prosthesis is configured to treat a mitral valve, the clamping arm is configured to capture and clamp an anterior valve leaflet when the valve clamping and fixing device is in a first form, and a connection point between the anchoring ring and the valve stent is configured to be located in a posterior valve leaflet region when the valve clamping and fixing device is in the first form.

8. The heart valve prosthesis capable of anchoring to a native valve leaflet according to claim 1, wherein the clamping mechanism comprises a first clamping arm and a second clamping arm, the anchoring ring is connected to the first clamping arm and the second clamping arm, and the first clamping arm and the second clamping arm are configured to capture and clamp different native valve leaflets respectively.

9. The heart valve prosthesis capable of anchoring to a native valve leaflet according to claim 8, wherein the valve clamping and fixing device further comprises a control filament, the control filament is detachably connected to the first clamping arm and the second clamping arm, and the control filament is configured to be pulled to enable the first clamping arm and the second clamping arm to open to capture the native valve leaflet.

10. The heart valve prosthesis capable of anchoring to a native valve leaflet according to claim 1, wherein the clamping mechanism comprises a plurality of clamping arms, wherein connection points between the anchoring ring and the plurality of clamping arms are at different heights, so that the anchoring ring is in a wavy shape.

11. The heart valve prosthesis capable of anchoring to a native valve leaflet according to claim 1, wherein a distal end of the valve stent is provided with a plurality of auxiliary support stems, and the plurality of auxiliary support stems are configured to be located in an atrium after the valve stent is mounted in place, and at least part of the plurality of auxiliary support stems are configured to fit against an atrial wall after the valve stent is mounted in place.

12. The heart valve prosthesis capable of anchoring to a native valve leaflet according to claim 1, wherein the heart valve prosthesis further comprises a sealing device, wherein the sealing device comprises an adaptive segment and an abutment segment, wherein the adaptive segment comprises a first arc segment, a second arc segment, and a leak-proof membrane, wherein the first arc segment is connected to the valve stent, the valve stent provides a fulcrum for the first arc segment, at least part of the first arc segment is configured to fit against an atrial wall after the valve stent is mounted in place, and the first arc segment is configured to rotate about the fulcrum toward a center of the valve stent to drive the second arc segment to rotate in a same direction as the first arc segment rotates when the atrial wall squeezes the first arc segment.
